# EUROPEAN PATENT APPLICATION

(11) **EP 4 512 397 A2**
(43) Date of publication of application: **26.02.2025**
(21) Application number: 24206614.0
(22) Date of filing: 26.08.2014
(51) Int. Cl.: A61K 31/225

(54) **NEW USE OF A COMBINATION OF AN ANGIOTENSIN RECEPTOR BLOCKER (ARB) WITH A NEUTRAL ENDOPEPTIDASE INHIBITOR (NEPI)**

(30) Priority: 26.08.2013 WO PCT/US2013/056680; 31.03.2014 US 201461972933 P
(62) Divisional of application: 23202190.7
(71) Applicant: Novartis AG, 4056 Basel (CH)
(72) Inventor: SHI, Victor Chengwei, New Jersey (US); LEFKOWITZ, Martin, New Jersey (US); RIZKALA, Adel Remond, New Jersey (US)
(74) Representative: Carpmaels & Ransford LLP

(57) **Abstract**

The present invention relates to methods and pharmaceutical compositions for the prevention or in delaying time to first occurrence of mortality, in particular cardiovascular death, and/or of cardiovascular hospitalizations in a patient suffering from chronic systolic heart failure, comprising administration of a therapeutically effective amount, or a prophylactically effective amount, of an Angiotensin Receptor Neprilysin inhibitor (ARNi) or of a combination of an Angiotensin Receptor Blocker (ARB) with a Neutral Endopeptidase inhibitor (NEPi) or with a NEPi pro-drug to said patient.

## Description

The present invention relates to methods and pharmaceutical compositions for the prevention or in delaying time to first occurrence of mortality, in particular cardiovascular death, and/or of cardiovascular hospitalizations in a patient suffering from chronic systolic heart failure, comprising administration of a therapeutically effective amount, or a prophylactically effective amount, of an Angiotensin Receptor Neprilysin inhibitor (ARNi) or of a combination of an Angiotensin Receptor Blocker (ARB) with a Neutral Endopeptidase inhibitor (NEPi) or with a NEPi pro-drug to said patient.

### Background of the Invention

Chronic heart failure (CHF) is a major public health problem characterized by significant mortality, frequent hospitalization, and poor quality of life, with an overall prevalence that is increasing throughout the world. In the United States (US) alone, approximately 5 million patients have heart failure (HF) and there are over half a million newly diagnosed cases annually. In Europe, the prevalence of HF is between 2 and 3%, and that in the elderly is estimated between 10 to 20%. HF is responsible for more hospitalizations than all forms of cancer combined and is the leading cause of hospitalization in patients older than 65 years of age. In-hospital mortality is excessive and readmission is distressingly common. Frequent hospitalizations, along with other direct and indirect costs, also place an enormous financial burden on healthcare systems.

A complex clinical syndrome with varying pathophysiological and clinical manifestations, HF is exhibited in patients with reduced left ventricular ejection fraction (LVEF) as well as preserved LVEF. Therapies targeted at improving outcomes in HF with a low EF have been well studied over the past two decades, leading to an improvement in survival as well as a decrease in morbidity, mostly in the form of decrease in re-hospitalization for HF, with angiotensin converting enzyme (ACE) inhibitors, angiotensin receptor blockers (ARBs), β-blockers and mineralocorticoid antagonists. For example, the Valsartan Heart Failure Trial (Val-HeFT), a randomized, placebo-controlled, double-blind, parallel-group trial [N Engl J Med 2001; 345:1667-1675], showed the following: The primary outcomes were mortality and the combined end point of mortality and morbidity, defined as the incidence of cardiac arrest with resuscitation, hospitalization for heart failure, or receipt of intravenous inotropic or vasodilator therapy for at least four hours. Overall mortality was similar in the two groups. The incidence of the combined end point (), however, was 13.2 percent lower with valsartan than with placebo (relative risk, 0.87; 97.5 percent confidence interval, 0.77 to 0.97; P=0.009), predominantly because of a lower number of patients hospitalized for heart failure: 455 (18.2 percent) in the placebo group and 346 (13.8 percent) in the valsartan group (P<0.001). Treatment with valsartan also resulted in significant improvements in NYHA class, ejection fraction, signs and symptoms of heart failure, and quality of life as compared with placebo (P<0.01). In a post hoc analysis of the combined end point and mortality in subgroups defined according to base-line treatment with angiotensin-converting-enzyme (ACE) inhibitors or beta-blockers, valsartan had a favorable effect in patients receiving neither or one of these types of drugs but an adverse effect in patients receiving both types of drugs.

However, despite advances in pharmacological and device therapies, the outlook remains poor. Overall 50% of patients die within 4 years, and 40% of patients admitted to hospital with HF die or are readmitted within 1 year. Thus, HF still represents a major cause of cardiac mortality and morbidity with a clear need for better therapy.

The neurohormonal response to cardiac injury is pivotal to the development and progression of heart failure. While increases in renin-angiotensin-aldosterone system (RAAS) and sympathetic nervous system (SNS) activity are believed to be harmful in the long-term, the increases in natriuretic peptides (NPs) are believed to be a beneficial compensatory response. As well as counteracting the effects of both RAAS and SNS, NPs have direct vasodilator and natriuretic actions, and exhibit antiproliferative and anti hypertrophic effects, thereby, potentially, ameliorating many of the pathophysiological abnormalities in HF.

Therefore, therapy that increases circulating concentrations of NPs through inhibition of neprilysin (neutral endopeptidase 24.11, NEP), the enzyme responsible for breaking down NPs, is considered an attractive therapeutic approach for a number of cardiovascular (CV) diseases, such as hypertension (HTN) and HF. However, results from previous clinical studies indicated that the effects of NEP inhibition alone are not sustained in the longer term. For example, NEP inhibition alone failed to demonstrate clinically meaningful blood pressure. It has been hypothesized that the vasoconstriction induced by ancillary properties of NEP inhibitors on other physiological substrates (e.g., angiotensin II) and feedback mechanisms can offset the BP lowering effect of ANP. Therefore, inhibition of NEP activity must be accompanied by inhibition of RAAS activity to achieve a beneficial effect.

Vasopeptidase inhibitors constitute a class of drugs that simultaneously inhibit both NEP and ACE. Indeed, omapatrilat, the most extensively evaluated agent from this class, demonstrated greater BP lowering as compared to other therapeutic classes, including ACE inhibitors (ACEls) and calcium channel blockers (CCBs). Additionally, omapatrilat also showed an encouraging trend toward reducing mortality and morbidity in patients with HF relative to enalapril. Unfortunately, omapatrilat treatment was associated with an increased incidence of angioedema compared with enalapril (2.17% vs. 0.68%) in hypertensive patients, though the increase in angioedema risk was less (0.8% and 0.5%, respectively) in HF patients. Due to this side effect of angioedema, omapatrilat was never approved for treatment of hypertension or heart failure patients.

These findings have led to the development of a novel class of drugs that combines the actions of NEP inhibitors and ARBs, known as angiotensin receptor blockade with neutral endopeptidase inhibition (ARNi) foreseen for the treatment of heart failure and hypertension. These innovative agents are aimed to better control blood pressure (BP) and also have a therapeutic potential in the setting of HF without increasing the risk of angioedema, which is commonly seen in case of concomitant inhibition of neprilysin and of ACE as mentioned above.

The compounds and pharmaceutical compositions disclosed herein include novel drug candidates potentially useful for the treatment of hypertension and/or heart failure. Such compounds or pharmaceutical compositions have been previously disclosed in WO 2003/059345, WO 2007/056546, WO 2009/061713 as well as WO2014029848, which are herein incorporated by reference.

The applicant has now clinically proven that administration of such an ARNI in addition to conventional HF treatment indeed provides a significant improvement in reducing cardiovascular mortality and morbidity in patients suffering from chronic systolic heart failure with reduced ejection fraction.

### Summary of the invention

Surprisingly, the administration of a pharmaceutical composition comprising a therapeutically effective amount, or a prophylactically effective amount, of an Angiotensin Receptor Neprilysin inhibitor (ARNi) as defined herein, or of a therapeutically effective amount, or a prophylactically effective amount, of a combination of an Angiotensin Receptor Blocker (ARB) with a Neutral Endopeptidase inhibitor (NEPi) or with a NEPi pro-drug, in a 1:1 molar ratio, as defined herein, to patients in need thereof has shown to significantly prevent or delay time to first occurrence of mortality, in particular of cardiovascular mortality, and/or of cardiovascular hospitalizations, in particular hospitalizations for heart failure, in patients suffering from chronic systolic heart failure, in particular in such patients with reduced ejection fraction.

The effective prevention or delaying time to first occurrence of mortality, in particular of cardiovascular mortality, and/or of cardiovascular hospitalizations, in particular hospitalizations for heart failure, in a patient suffering from chronic systolic heart failure with reduced ejection fraction was shown by administration of said pharmaceutical composition in comparison to the current standard of care treatment with an ACE inhibitor, namely enalapril, administered twice daily at a dose of 10 mg.

Said pharmaceutical composition comprises
a) a therapeutically effective amount of the compound of the formula (I)

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in the anion form;
   A₂ is (2R,4S)-5-biphenyl-4-yl-4-(3-carboxy-propionylamino)-2-methyl-pentanoic acid ethyl ester in the anion form;
   Na is a sodium ion;
   y is 1 to 3; and
   x is 0 to 3;
      or
b) a combination comprising a therapeutically effective amount of a 1:1 molar ratio
   (i) valsartan or a pharmaceutically acceptable salt thereof; and
   (ii) sacubitril or a pharmaceutically acceptable salt thereof.

Sacubitril is (2R,4S)-5-biphenyl-4-yl-4-(3-carboxy-propionylamino)-2-methyl-pentanoic acid ethyl ester, also named N-(3-carboxy-1-oxopropyl)-(4S)-(p-phenylphenylmethyl)-4-amino-2R-methylbutanoic acid ethyl ester.

In one embodiment y is 1, 2 or 3, preferably 3.

In one embodiment x is selected from 0, 0.25, 0.5, 0.75, 1, 1.25, 1.5, 1.74, 2, 2.25, 2.5, 2.75 and 3, preferably 0, 0.5 and 2.5.

In one embodiment, the patient is a human patient.

In one embodiment, the compound of formula (I) is used.

In another embodiment thereof, the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In one embodiment, the patient suffering from chronic systolic heart failure, in particular a patient with chronic systolic heart failure with reduced ejection fraction, has at least one of the following characteristics:
i) heart failure of NYHA class II, III or IV,
ii) an elevated plasma BNP or NT-proBNP level, preferably a plasma BNP ≥100 pg/mL (or NT-proBNP ≥400 pg/mL), more preferably a plasma BNP ≥150 pg/mL or NT-proBNP ≥600 pg/mL, and
iii) a reduced left ventricular ejection fraction (LVEF) of ≤40%, preferably ≤35%.

In addition, the patient might be characterized by one or more of the following:
iv) prior hospitalization for heart failure within the last 12 months,
v) a stable ACE inhibitor or ARB at dose ≥ enalapril 10 mg daily + beta-blocker (unless contraindicated or intolerant) + aldosterone antagonist (as indicated),
vi) systolic blood pressure ≥ 95 mm Hg,
vii) eGFR ≥ 30 ml/min/1.73 m² and
viii) serum K ≤ 5.4 mEq/L.

In another embodiment, the present invention is directed to the pharmaceutical composition as defined above for preventing of or delaying time to first occurrence of mortality, in particular of cardiovascular mortality, and/or of cardiovascular hospitalizations, in particular hospitalizations for heart failure, in a patient suffering from chronic systolic heart failure, in particular chronic systolic heart failure with reduced ejection fraction.

In another embodiment, the present invention is directed to the use of the pharmaceutical composition as defined above for the preparation of a medicament for the prevention or for delaying time to first occurrence of mortality, in particular of cardiovascular mortality, and/or of cardiovascular hospitalizations, in particular hospitalizations for heart failure, in a patient suffering from chronic systolic heart failure, in particular chronic systolic heart failure with reduced ejection fraction.

### Definitions

Throughout this specification and in the claims that follow, the following terms are defined with the following meanings, unless explicitly stated otherwise.

The term "prevention" refers to prophylactic administration to a healthy subject to prevent the development of the conditions mentioned herein. Moreover, the term "prevention" means prophylactic administration to patients being in a pre-stage of the conditions to be treated.

The term "treatment" is understood the management and care of a patient for the purpose of combating the disease, condition or disorder.

The term "therapeutically effective amount" refers to an amount of a drug or a therapeutic agent that will elicit the desired biological and/or medical response of a tissue, system or an animal (including man) that is being sought by a researcher or clinician.

The terms "patient" include, but are not limited to, humans, dogs, cats, horses, pigs, cows, monkeys, rabbits and mice. The preferred patients are humans.

The terms "administration of" and or "administering a" compound should be understood to mean providing a compound of the invention or a pharmaceutically acceptable salt or ester thereof, or a pro-drug thereof to a subject in need of treatment. The administration of the composition of the present invention in order to practice the present methods of therapy is carried out by administering a therapeutically effective amount of the compounds in the composition to a subject in need of such treatment or prophylaxis. The need for a prophylactic administration according to the methods of the present invention is determined via the use of well-known risk factors. The effective amount of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgment.

The term "prophylactically effective amount" as used herein means the amount of the active compounds in the composition that will elicit the biological or medical response in a tissue, system, subject, or human that is being sought by the researcher, veterinarian, medical doctor or other clinician, to prevent the onset of a disease characterized and / or manifested by atrial enlargement and/or remodeling.

The term "pharmaceutically acceptable", as used herein, refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

The term "eGFR" refers to estimated glomerular filtration rate. Within the context of the present invention the eGFR is calculated by the Modification in Diet in Renal Disease (MDRD) formula, which is the one recommended by NICE and The Renal Association (UK), and which is based on the equation described in Levey AS, Bosch JP, Lewis JB, et al; "A more accurate method to estimate glomerular filtration rate from serum creatinine: a new prediction equation. Modification of Diet in Renal Disease Study Group." Ann Intern Med. 1999 Mar 16;130(6):461-70.

The New York Heart Association (NYHA) classification grades the severity of heart failure symptoms as one of four functional classes. The NYHA classification is widely used in clinical practice and in research because it provides a standard description of severity that can be used to assess response to treatment and to guide management. The New York Heart Association functional classification based on severity of symptoms and physical activity:
Class I: No limitation of physical activity. Ordinary physical activity does not cause undue breathlessness, fatigue, or palpitations.
Class II: Slight limitation of physical activity. Comfortable at rest, but ordinary physical activity results in undue breathlessness, fatigue, or palpitations.
Class III: Marked limitation of physical activity. Comfortable at rest, but less than ordinary physical activity results in undue breathlessness, fatigue, or palpitations.
Class IV: Unable to carry on any physical activity without discomfort. Symptoms at rest can be present. If any physical activity is undertaken, discomfort is increased.

Choice of endpoints: Cardiovascular death and heart failure hospitalization both reflect disease-specific endpoints related to progressive worsening of the heart failure syndrome, and experienced by patients with systolic heart failure. These endpoints can be modified by treatments improving this condition, which has generally proved to be the case with drugs such as ACEls, aldosterone antagonists, and β-blockers as well as devices for cardiac resynchronization therapy.

### Detailed Description of the Invention

### Methods of treatment

The present invention is based upon the surprising and unexpected finding that certain drugs (i.e. LCZ696) presumed to be effective for the treatment of cardiovascular disease or conditions, such as heart failure or hypertension, in human subjects have now been shown to significantly prevent or delay time to first occurrence of mortality, in particular of cardiovascular mortality, and/or of cardiovascular hospitalizations, in particular hospitalizations for heart failure, in patients suffering from chronic systolic heart failure, in particular heart failure with reduced ejection fraction, in comparison to the current standard of care treatment with an ACE inhibitor, namely enalapril administered twice daily at a dose of 10 mg.

The significantly improved effectiveness in delaying time to occurrence of mortality, in particular of cardiovascular mortality, and/or in the prevention of or delaying time to first occurrence of cardiovascular hospitalizations, in particular hospitalizations for heart failure, in a patient suffering from chronic systolic heart failure, in particular heart failure with reduced ejection fraction, was shown by administration of said drug in comparison to the current standard of care treatment with an ACE inhibitor, namely enalapril, administered twice daily at a dose of 10 mg. This comparison took place in the form of a large randomized clinical trial, named PARADIGM (for details see Example section).

The trial showed that LCZ696 treatment reduced the risk of cardiovascular death and the risk of hospitalization for heart failure by 20% compared to ACE inhibition with enalapril in patients with chronic heart failure and reduced ejection fraction (HR 0.80; 95% Cl, 0.73-0.87; p<0.0001). The difference in favor of LCZ696 occurred early in the trial and was observed at each interim analysis.
- The primary outcome, i.e. cardiovascular death and hospitalization for heart failure, occurred in 914 patients (21.8%) in the LCZ696 group and 1117 patients (26.5%) in the enalapril group.
- There were 558 (13.3%) and 693 deaths (16.5%) due to cardiovascular causes in the LCZ696 and enalapril treatment groups, respectively, (HR 0.80; 95% Cl, 0.71 to 0.89; P<0.0001).
- There were 537 (12.8%) patients hospitalized for heart failure in the LCZ696 treatment group compared with 658 patients (15.6%) in the enalapril group (hazard ratio 0.79; 95% Cl, 0.71 to 0.89; P<0.0001).

Thus, the invention encompasses a method for preventing or delaying time to first occurrence of mortality, in particular of cardiovascular mortality, cardiovascular hospitalizations, in particular hospitalizations for heart failure or the combination thereof in a patient suffering from chronic systolic heart failure, in particular heart failure with reduced ejection fraction, comprising administering to said patient in need thereof a therapeutically effective amount, or a prophylactically effective amount, of an Angiotensin Receptor Neprilysin inhibitor (ARNi) as defined herein or of a therapeutically effective amount, or a prophylactically effective amount, of a combination of an Angiotensin Receptor Blocker (ARB) with a Neutral Endopeptidase inhibitor (NEPi) or with a NEPi pro-drug, in a 1:1 molar ratio, as defined herein.

The invention encompasses a method for reducing the rate of cardiovascular death or heart failure hospitalization or a combination thereof in patients with chronic heart failure with systolic dysfunction, comprising administering to said patient in need thereof a therapeutically effective amount, or a prophylactically effective amount, of an Angiotensin Receptor Neprilysin inhibitor (ARNi) as defined herein or of a therapeutically effective amount, or a prophylactically effective amount, of a combination of an Angiotensin Receptor Blocker (ARB) with a Neutral Endopeptidase inhibitor (NEPi) or with a NEPi pro-drug, in a 1:1 molar ratio, as defined herein.

The invention also encompasses a method for the treatment of chronic heart failure in patient with systolic dysfunction, comprising administering to said patient in need thereof a therapeutically effective amount, or a prophylactically effective amount, of an Angiotensin Receptor Neprilysin inhibitor (ARNi) as defined herein or of a therapeutically effective amount, or a prophylactically effective amount, of a combination of an Angiotensin Receptor Blocker (ARB) with a Neutral Endopeptidase inhibitor (NEPi) or with a NEPi pro-drug, in a 1:1 molar ratio, as defined herein, to patients in need thereof, wherein the treatment had been shown to reduce the rate of cardiovascular death and/or heart failure hospitalization, in particular when compared to enalapril, in patients with chronic heart failure with systolic dysfunction.

In one embodiment, the comparison of the ARNI to enalapril was carried out in a clinical trial setting, namely the PARADIGM trial (see Example section for more details).

In the context of the present invention, said Angiotensin Receptor Neprilysin inhibitor (ARNi) is
a) a therapeutically effective amount of the compound of the formula (I)

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in the anion form;
   A₂ is (2R,4S)-5-biphenyl-4-yl-4-(3-carboxy-propionylamino)-2-methyl-pentanoic acid ethyl ester in the anion form;
   Na is a sodium ion;
   y is 1 to 3; and
   x is 0 to 3.

In the context of the present invention, said combination of an Angiotensin Receptor Blocker (ARB) with a Neutral Endopeptidase inhibitor (NEPi) or with a NEPi pro-drug, in a 1:1 molar ratio is
b) a combination comprising a therapeutically effective amount of a 1:1 molar ratio of
(i) valsartan or a pharmaceutically acceptable salt thereof; and
(ii) sacubitril or a pharmaceutically acceptable salt thereof.

In another embodiment, the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In one embodiment, the patient is a human patient.

In one embodiment, the patient suffering from chronic systolic heart failure, in particular the patient with chronic systolic heart failure with reduced ejection fraction, has at least one of the following characteristics:
i) heart failure of NYHA class II, III or IV,
ii) an elevated plasma BNP or NT-proBNP level, preferably a plasma BNP ≥100 pg/mL (or NT-proBNP ≥400 pg/mL), more preferably a plasma BNP ≥150 pg/mL or NT-proBNP ≥600 pg/mL, and
iii) a reduced left ventricular ejection fraction (LVEF) of ≤40%, preferably ≤35%.

In addition, the patient might be characterized by one or more of the following:
iv) prior hospitalization for heart failure within the last 12 months,
v) a stable ACE inhibitor or ARB at dose ≥ enalapril 10 mg daily + beta-blocker (unless contraindicated or intolerant) + aldosterone antagonist (as indicated),
vi) systolic blood pressure ≥ 95 mm Hg,
vii) eGFR ≥ 30 ml/min/1.73 m² and
viii) serum K ≤ 5.4 mEq/L.

In one embodiment, the patient has heart failure classified as NYHA class II, III or IV and has systolic dysfunction. In another embodiment the patient has heart failure classified as NYHA class II. In a further embodiment, the patient has heart failure classified as NYHA class II with systolic dysfunction and has a reduced left ventricular ejection fraction (LVEF) of ≤35%.

In one embodiment, the present invention provides that the therapeutically effective amount of compound of formula (I) or of the combination is effective to prevent or delay time to first occurrence of cardiovascular hospitalization, preferably wherein the cardiovascular hospitalization is hospitalization for heart failure, non-fatal myocardial infarction, non-fatal stroke, or resuscitated sudden death, in particular hospitalization for heart failure, more particularly first hospitalization for worsening heart failure.

In another embodiment, the present invention provides that the therapeutically effective amount of compound of formula (I) or of the combination is effective to prevent or delay time to first occurrence of mortality, in particular cardiovascular mortality.

In another embodiment the mortality is a sudden death.

In a further embodiment, the therapeutically effective amount of compound of formula (I) or of the combination has been shown to reduce the rate of cardiovascular death, or to reduce the rate of heart failure hospitalization or to reduce the rate of cardiovascular death and the rate of heart failure hospitalization.

In one embodiment, the therapeutically effective amount of compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696) has been shown to reduce the rate of cardiovascular death or of heart failure hospitalization or of a combination thereof.

In another embodiment, the therapeutically effective amount of compound of formula (I) or of the combination has been shown to reduce the rate of cardiovascular death and heart failure hospitalization in patients with chronic heart failure classified as NYHA class II, III or IV with systolic dysfunction, preferably wherein the patient also has a reduced left ventricular ejection fraction (LVEF) of ≤35%.

In one embodiment, the therapeutically effective amount of compound of formula (I) or of the combination has been shown to reduce the rate of all-cause mortality.

In a further embodiment, the patient receives a base treatment with a stable dose of a beta-blocker, an aldosterone antagonists, and/ or a diuretic.

In one embodiment thereof, the patient receives base treatment with a stable dose of a beta-blocker and optionally an aldosterone antagonist.

In one embodiment, the patient receives base treatment with a stable dose of a beta-blocker.

The present invention also provides that the therapeutically effective amount of compound of formula (I) or of the combination is also effective to prevent or delay time to first occurrence of new onset atrial fibrillation.

In a further embodiment, the present invention also provides that the therapeutically effective amount of compound of formula (I) or of the combination is also effective to prevent or slow decline in estimated glomerular filtration rate (eGFR).

The present invention also provides that the therapeutically effective amount of compound of formula (I) or of the combination also reduces the blood pressure of the patient The present invention also provides that the therapeutically effective amount of compound of formula (I) or of the combination is more effective in preventing or delaying time to first occurrence of mortality, in particular cardiovascular mortality, and/or of cardiovascular hospitalizations, in particular hospitalization for heart failure, in patients suffering from chronic systolic heart failure, in particular heart failure with reduced ejection fraction, compared to a therapeutically effective amount of an ACE inhibitor.

In one embodiment, this ACE inhibitor is enalapril, in particular enalapril administered twice daily at a dose of 10 mg each.

In one embodiment, the administration of a therapeutically effective amount of compound of formula (I) or of the combination is at least 10% more effective with regard to preventing or delaying time to first occurrence of mortality, in particular cardiovascular mortality, and/or of cardiovascular hospitalizations, in particular hospitalization for heart failure, in patients suffering from chronic systolic heart failure, in particular heart failure with reduced ejection fraction, compared to a medicament comprising a therapeutically effective amount of an ACE inhibitor, in particular enalapril as defined above.

In another embodiment, the administration of a therapeutically effective amount of compound of formula (I) or of the combination is at least 15% more effective with regard to preventing or delaying time to first occurrence of mortality, in particular cardiovascular mortality, and/or of cardiovascular hospitalizations, in particular hospitalization for heart failure, in patients suffering from chronic systolic heart failure, in particular heart failure with reduced ejection fraction, than a medicament comprising a therapeutically effective amount of an ACE inhibitor, in particular enalapril as defined above.

According to one aspect of the present invention, the administration of a therapeutically effective amount of compound of formula (I) or of the combination is superior to enalapril in preventing or delaying time to first occurrence of mortality and/or of cardiovascular hospitalizations in patients suffering from chronic systolic heart failure with reduced ejection fraction.

According to another aspect of the invention, the administration of a therapeutically effective amount of compound of formula (I) or of the combination as defined herein is at least 10%, preferably at least 15% more effective than a medicament comprising a therapeutically effective amount of enalapril in reducing the rate of mortality, in particular cardiovascular death, and/or of cardiovascular hospitalizations, in particular hospitalizations for heart failure.

According to a separate aspect, the administration of a therapeutically effective amount of compound of formula (I) or of the combination as defined herein is statistically superior to a medicament comprising a therapeutically effective amount of enalapril in reducing the rate of cardiovascular death and/or of cardiovascular hospitalizations, in particular hospitalizations for heart failure.

In one embodiment thereof, the statistical superiority is expressed as a 20% risk reduction rate.

According to another aspect of the invention, the administration of a therapeutically effective amount of compound of formula (I) or of the combination as defined herein shows at least 20% risk reduction compared to a medicament comprising a therapeutically effective amount of enalapril with regard to the rate of cardiovascular death.

According to another aspect of the invention, the administration of a therapeutically effective amount of compound of formula (I) or of the combination as defined herein shows an at least 13% risk reduction compared to a medicament comprising a therapeutically effective amount of enalapril in reducing the rate of cardiovascular hospitalizations.

In one embodiment thereof, the administration of a therapeutically effective amount of compound of formula (I) or of the combination as defined herein shows an at least 20% risk reduction compared to a medicament comprising a therapeutically effective amount of enalapril in reducing the rate of hospitalizations for heart failure, more particularly with regard to the rate of first hospitalization for worsening heart failure.

According to another aspect of the invention, the administration of a therapeutically effective amount of compound of formula (I) or of the combination as defined herein shows an at least 15% risk reduction compared to a medicament comprising a therapeutically effective amount of enalapril in reducing the rate of all-cause mortality.

In another embodiment, the administration of a therapeutically effective amount of compound of formula (I) or of the combination as defined herein has been shown to improve the NYHA class of more patients taking the medicament than of patients taking a medicament comprising a therapeutically effective amount of enalapril.

In a further embodiment, the administration of a therapeutically effective amount of compound of formula (I) or of the combination as defined herein in comparison to a medicament comprising a therapeutically effective amount of enalapril has been shown to lead to at least one of the following:
- a greater decrease in NTproBNP levels
- a consistent elevation in cGMP levels, and
- a greater reduction in troponin levels.

In another aspect of the invention, the administration of a therapeutically effective amount of compound of formula (I) or of the combination as defined herein in comparison to a medicament comprising a therapeutically effective amount of enalapril showed a more potent blood pressure lowering effect.

In a further embodiment of the present invention the compound of formula (I) is used.

In a further embodiment thereof, the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In a further embodiment of the present invention a daily overall dose of LCZ696 from 50 mg to 400 mg is administered, taken in one or more separate intakes. In one embodiment thereof, LCZ696 is administered twice daily with a dose of 50 mg, 100 mg, or 200 mg.

In another embodiment of the invention, the patient is orally administered the combination as defined herein in two separate intakes (b.i.d.) per day, each intake comprising 103 mg valsartan, or a pharmaceutically acceptable salt thereof, and 97 mg sacubitril, or a pharmaceutically acceptable salt thereof, per dosage unit.

All the aforementioned embodiments for the methods of protection and treatment according to the present invention are equally applicable to
- the use of a compound of formula (I) or of the combination (i)/(ii) as defined herein for the manufacture of a medicament for use according to the present invention,
- the use of a compound of formula (I) or of the combination (i)/(ii) as defined herein according to the present invention,
- a compound of formula (I) or of the combination (i)/(ii) as defined herein for use according to the present invention,
- the pharmaceutical compositions comprising a compound of formula (I) or of the combination (i)/(ii) as defined herein for the use according to the present invention,
- the use of the pharmaceutical compositions comprising a compound of formula (I) or of the combination (i)/(ii) as defined herein according to the present invention and
- the use of the pharmaceutical compositions comprising a compound of formula (I) or of the combination (i)/(ii) as defined herein for the manufacture of a medicament for use according to the present invention.

Some of these aspects are further described in more detail below, but this description should not be construed as limiting.

### Use for the manufacture of a medicament

In another embodiment, the present invention is directed to the use of
a) a therapeutically effective amount of the compound of the formula (I)

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in the anion form;
   A₂ is (2R,4S)-5-biphenyl-4-yl-4-(3-carboxy-propionylamino)-2-methyl-pentanoic acid ethyl ester in the anion form;
   Na is a sodium ion;
   y is 1 to 3; and
   x is 0 to 3;
   or
b) a combination comprising a therapeutically effective amount of a 1:1 molar ratio
   (i) valsartan or a pharmaceutically acceptable salt thereof; and
   (ii) sacubitril or a pharmaceutically acceptable salt thereof,

for the preparation of a medicament for the prevention or delaying time to first occurrence of mortality, in particular of cardiovascular mortality, and/or of cardiovascular hospitalizations, in particular hospitalizations for heart failure, in a patient suffering from chronic systolic heart failure, in particular chronic systolic heart failure with reduced ejection fraction, or
for the preparation of a medicament for reducing the rate of cardiovascular death and/or heart failure hospitalization in patients with chronic heart failure with systolic dysfunction, in particular chronic systolic heart failure with reduced ejection fraction, or
for the preparation of a medicament for the treatment of chronic heart failure in a patient with systolic dysfunction, wherein the medicament has been shown to reduce the rate of cardiovascular death and/or heart failure hospitalization in patients with chronic heart failure with systolic dysfunction, in particular chronic systolic heart failure with reduced ejection fraction.

In an alternative embodiment, the invention is directed to the use of
a) a therapeutically effective amount of the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a therapeutically effective amount of a 1:1 molar ratio of
   (i) valsartan or a pharmaceutically acceptable salt thereof; and
   (ii) sacubitril or a pharmaceutically acceptable salt thereof,
for the preparation of a medicament for the treatment of chronic heart failure in a patient with systolic dysfunction, wherein the medicament has been shown to reduce the rate of cardiovascular death and/or heart failure hospitalization in patients with chronic heart failure with systolic dysfunction when compared to enalapril

In all the above embodiments, in a preferred aspect thereof the compound of formula (I) is used and is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In all the above embodiments, in a preferred aspect thereof the patients have chronic heart failure classified as NYHA class II, III or IV with systolic dysfunction.

In all the above embodiments, in a preferred aspect thereof, the patient also has a reduced left ventricular ejection fraction (LVEF) of ≤35%.

In another embodiment of the invention, there is an at least 20% risk reduction rate in comparison to enalapril.

In a further embodiment, the medicament also has been shown to reduce the rate of all-cause mortality.

### Pharmaceutical composition for use

In another embodiment, the present invention is directed to a pharmaceutical composition comprising:
a) the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in the anion form;
   A₂ is (2R,4S)-5-biphenyl-4-yl-4-(3-carboxy-propionylamino)-2-methyl-pentanoic acid ethyl ester in the anion form;
   Na is a sodium ion;
   y is 1 to 3; and
   x is 0 to 3;
   or
b) a combination of
   (i) valsartan or a pharmaceutically acceptable salt thereof; and
   (ii) sacubitril or a pharmaceutically acceptable salt thereof,
for the prevention or delaying time to first occurrence of mortality, in particular of cardiovascular mortality, and/or of cardiovascular hospitalizations, in particular hospitalizations for heart failure, in a patient suffering from chronic systolic heart failure, in particular chronic systolic heart failure with reduced ejection fraction.

In another embodiment, the present invention is directed to said pharmaceutical composition as defined herewithin for use in reducing the rate of cardiovascular death and/or heart failure hospitalization in patients with chronic heart failure with systolic dysfunction, in particular chronic systolic heart failure with reduced ejection fraction.

In a further embodiment, the invention is directed to said pharmaceutical composition as defined herewithin for the treatment of chronic heart failure in a patient with systolic dysfunction, wherein the medicament has been shown to reduce the rate of cardiovascular death and/or heart failure hospitalization in patients with chronic heart failure with systolic dysfunction, in particular wherein the patient has chronic systolic heart failure with reduced ejection fraction.

In another embodiment thereof, the invention is directed to said pharmaceutical composition as defined herewithin for the treatment of chronic heart failure in a patient with systolic dysfunction, wherein the medicament has been shown to reduce the rate of cardiovascular death and/or heart failure hospitalization in patients with chronic heart failure with systolic dysfunction when compared to enalapril.

In all the above embodiments, in a preferred aspect thereof, the patient has chronic heart failure classified as NYHA class II, III or IV. In another embodiment thereof, the patient also has a reduced left ventricular ejection fraction (LVEF) of ≤35%.

In a further embodiment thereof, the said pharmaceutical composition has been shown to reduce the rate of all-cause mortality.

In another embodiment thereof, there is an at least 20% risk reduction rate in comparison to enalapril.

In one embodiment, the pharmaceutical composition as referred to above comprises in addition one or more pharmaceutically acceptable carriers.

In one embodiment, the pharmaceutical composition comprises the compound of formula (I) which is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

### Compound or combination for use

In a separate aspect, the present invention is directed to
a) the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a 1:1 molar ratio of
   (i) valsartan or a pharmaceutically acceptable salt thereof; and
   (ii) sacubitril or a pharmaceutically acceptable salt thereof,
for use in the prevention or delaying time to first occurrence of mortality, in particular of cardiovascular mortality, and/or of cardiovascular hospitalizations, in particular hospitalizations for heart failure, in a patient suffering from chronic systolic heart failure, in particular chronic systolic heart failure with reduced ejection fraction.

In another embodiment, the present invention is directed to
a) the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a 1:1 molar ratio of
   (i) valsartan or a pharmaceutically acceptable salt thereof; and
   (ii) sacubitril or a pharmaceutically acceptable salt thereof,
for use in reducing the rate of cardiovascular death and/or heart failure hospitalization in patients with chronic heart failure with systolic dysfunction, in particular chronic systolic heart failure with reduced ejection fraction.

In a further embodiment, the present invention is directed to
a) the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a 1:1 molar ratio of
   (i) valsartan or a pharmaceutically acceptable salt thereof; and
   (ii) sacubitril or a pharmaceutically acceptable salt thereof,
for the treatment of chronic heart failure in a patient with systolic dysfunction, wherein the medicament has been shown to reduce the rate of cardiovascular death and/or heart failure hospitalization in patients with chronic heart failure with systolic dysfunction, in particular wherein the patient has chronic systolic heart failure with reduced ejection fraction.

In another embodiment, the present invention is directed to
a) the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a 1:1 molar ratio of
   (i) valsartan or a pharmaceutically acceptable salt thereof; and
   (ii) sacubitril or a pharmaceutically acceptable salt thereof,
for the treatment of chronic heart failure in a patient with systolic dysfunction, wherein the medicament has been shown to reduce the rate of cardiovascular death and/or heart failure hospitalization in patients with chronic heart failure with systolic dysfunction when compared to enalapril

In any of the above embodiments directed to (a) the compound of the formula (I) or (b) the combination comprising valsartan and sacubitril, as defined above for use, the following further embodiments might apply:
- the patient has chronic heart failure classified as NYHA class II, III or IV,
- the patient also has a reduced left ventricular ejection fraction (LVEF) of ≤35%,
- the compound or combination has been shown to reduce the rate of all-cause mortality,
- there is an at least 20% risk reduction rate in comparison to enalapril.

In any of the above embodiments, the compound of the formula (I) is a preferred embodiment; in particular the compound of formula (I) which is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

Accordingly, in one aspect, the present invention relates to the compound trisodium [3-((1S,3R)-1-biphenyl-4-ylmethyl-3-ethoxycarbonyl-1-butyl carbamoyl)propionate-(S)-3'-methyl-2'-(pentanoyl{2"-(tetrazol-5-ylate)biphenyl-4'-ylmethyl}amino)butyrate] hemipentahydrate for the prevention of or delaying time to first occurrence of mortality and/or of cardiovascular hospitalizations in a patient with chronic systolic heart failure. In another aspect, the present invention relates to the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate for reducing the rate of cardiovascular death and/or heart failure hospitalization in patients with chronic heart failure with systolic dysfunction.

In another aspect, the present invention relates to the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate for the treatment of chronic heart failure in a patient with systolic dysfunction, wherein the medicament has been shown to reduce the rate of cardiovascular death and/or heart failure hospitalization, in particular in comparison to enalapril.

In another aspect, the present invention relates to the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate for reducing the rate of cardiovascular death and/or heart failure hospitalization in patients with chronic heart failure with systolic dysfunction compared to enalapril.

In one embodiment of the above aspects, the patient has chronic heart failure classified as NYHA class II, III or IV. In another embodiment thereof, the patient also has a reduced left ventricular ejection fraction (LVEF) of ≤35%.

In a further embodiment thereof, compound has been shown to reduce the rate of all-cause mortality.

In a further embodiment thereof, the compound has shown at least 20% risk reduction compared to a medicament comprising a therapeutically effective amount of enalapril with regard to the rate of cardiovascular death.

### Compounds and combinations and pharmaceutical compositions for use according to the invention

The compounds of the invention used in the aforementioned methods are
a) a compound of the formula (I)

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in the anion form;
   A₂ is (2R,4S)-5-biphenyl-4-yl-4-(3-carboxy-propionylamino)-2-methyl-pentanoic acid ethyl ester in the anion form;
   Na is a sodium ion;
   y is 1 to 3, preferably 1, 2, or 3; and
   x is 0 to 3, preferably 0, 0.5, 1, 1.5, 2, 2.5, or 3;
   or
b) a combination comprising a 1:1 molar ratio of
   (i) valsartan or a pharmaceutically acceptable salt thereof; and
   (ii) of sacubitril or a pharmaceutically acceptable salt thereof.

In one embodiment, y is 3 and x is 2.5.

In another embodiment, y is 3 and x is 0.5.

Sacubitril is the INN for N-(3-carboxy-1-oxopropyl)-(4S)-(p-phenylphenylmethyl)-4-amino-2R-methylbutanoic acid ethyl ester. This is a prodrug for (2R,4S)-5-biphenyl-4-yl-4-(3-carboxy-propionyl amino)-2-methyl-pentanoic acid.

In another embodiment, the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In a preferred embodiment, the compound of formula (I) is present in crystalline form.

In another embodiment, the combination comprises a 1:1 molar ratio
(i) of valsartan; and
(ii) of sacubitril or a pharmaceutically acceptable salt thereof, such as sodium or calcium salt.

In a preferred embodiment, the invention encompasses a pharmaceutical composition for use in the prevention or delaying time to first occurrence of mortality, in particular cardiovascular mortality, and/or of cardiovascular hospitalizations, in particular hospitalization for heart failure, in patients suffering from chronic systolic heart failure, in particular heart failure with reduced ejection fraction in a patient as described herein, the composition comprising a therapeutically effective amount of trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (Compound LCZ696). Such compounds and pharmaceutical compositions have been previously disclosed in WO2007/056546 and WO 2009/061713, whose preparative teachings are incorporated herein by reference.

In a further embodiment of the invention, the pharmaceutical compositions for use according to the present invention comprise trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate] hemipentahydrate (LCZ696) and deliver upon administration the NEP inhibitor pro-drug sacubitril and the angiotensin receptor blocker valsartan together to the patient.

The pharmaceutical compositions for use according to the present invention comprise a therapeutically effective amount of a compound of the formula (I) or of a combination of valsartan and sacubitril as defined herein above or a pharmaceutically acceptable salt or ester thereof, or pro-drug thereof. Each dosage unit can contain the daily dose or may contain a fraction of the daily dose, such as one-half or one-third of the dose.

In one embodiment of the invention for all of its uses, the pharmaceutical composition comprises the the NEP inhibitor pro-drug sacubitril, namely N-(3-carboxy-1-oxopropyl)-(4S)-p-phenylphenylmethyl)-4-amino-(2R)-methylbutanoic acid ethyl ester or the NEP inhibitor N-(3-carboxy-1-oxopropyl)-(4S)-p-phenylphenylmethyl)-4-amino-(2R)-methylbutanoic acid, or pharmaceutically acceptable salts thereof, and the Angiotensin Receptor Blocker valsartan or a pharmaceutically acceptable salt thereof. Such combinations are for example disclosed within international patent application WO 2003/059345, which is herewith incorporated by reference.

In one embodiment, the pharmaceutical composition comprises the NEP inhibitor pro-drug sacubitril, namely N-(3-carboxy-1-oxopropyl)-(4S)-p-phenylphenylmethyl)-4-amino-(2R)-methylbutanoic acid ethyl ester or the NEP inhibitor N-(3-carboxy-1-oxopropyl)-(4S)-p-phenylphenylmethyl)-4-amino-(2R)-methylbutanoic acid, or pharmaceutically acceptable salts thereof, and the Angiotensin Receptor Blocker valsartan or a pharmaceutically acceptable salt thereof, in a 1:1 molar ratio.
(i) Valsartan or (S)-N-valeryl-N-{[2'-(1H-tetrazole-5-yl)-biphenyl-4-yl]-methyl}-valine) or a pharmaceutically acceptable salt thereof that can be purchased from commercial sources or can be prepared according to known methods, such as described in U.S. Patent No. 5,399,578 and EP 0443983, whose preparative teachings are incorporated by reference herein. Valsartan may be used in certain embodiments of the invention in its free acid form, as well as in any suitable salt form. Depending upon the circumstance, esters or other derivatives of the carboxylic grouping may be employed as well as salts and derivatives of the tetrazole grouping.
(ii) sacubitril, namely N-(3-carboxy-1-oxopropyl)-(4S)-(p-phenylphenylmethyl)-4-amino-2R-methylbutanoic acid ethyl ester, or (2R,4S)-5-biphenyl-4-yl-4(3-carboxy-propionyl amino)-2-methyl-pentanoic acid can be prepared by known methods such as described in U.S.

Patent No. 5,217,996 which is herein incorporated by reference.

Either compound may be admixed with valsartan to prepare compounds of the formula (i)/(ii).

The corresponding active ingredient or a pharmaceutically acceptable salt thereof may also be used in the form of a hydrate or include other solvents used for crystallization.

Preferably, compound (I) or LCZ696, or compounds (i)/(ii) are substantially pure or in a substantially pure form. As used herein, "substantially pure" refers to at least about 90% purity, more preferably at least about 95% and most preferably at least about 98% purity.

Also preferred is that compound (I) or L, or compounds (i)/(ii) are solid or a solid form or solid state. The solid, solid form or solid state can be crystalline, partially crystalline, amorphous or polymorphous, preferably in the crystalline form.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application.

The pharmaceutical preparations of the invention contain, for example, from about 0.1% to about 100%, e. g. 80% or 90%, or from about 1% to about 60%, of the active ingredient. The term "about" or "approximately", as used herein in each instance, shall have the meaning of within 10%, more preferably within 5%, of a given value or range.

Pharmaceutical preparations according to the invention for enteral or parenteral administration are, e.g., those in unit dose forms, such as sugar-coated tablets, tablets, capsules, bars, sachets, granules, syrups, aqueous or oily suspensions or suppositories and furthermore ampoules. These are prepared in a manner known per se, e. g. by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

Tablets may be formed from the active compound with fillers, for example calcium phosphate; disintegrating agents, for example maize starch, lubricating agents, for example magnesium stearate; binders, for example microcrystalline cellulose or polyvinylpyrrolidone and other optional ingredients known in the art to permit tabletting the mixture by known methods. Similarly, capsules, for example hard or soft gelatin capsules, containing the active compound with or without added excipients, may be prepared by known methods. The contents of the capsule may be formulated using known methods so as to give sustained release of the active compound.

Other dosage forms for oral administration include, for example, aqueous suspensions containing the active compound in an aqueous medium in the presence of a non-toxic suspending agent such as sodium carboxymethylcellulose, and oily suspensions containing the active compounds in a suitable vegetable oil, for example arachis oil.

The active compound may be formulated into granules with or without additional excipients. The granules may be ingested directly by the patient or they may be added to a suitable liquid carrier (e.g. water) before ingestion. The granules may contain disintegrants, e.g. an effervescent pair formed from an acid and a carbonate or bicarbonate salt to facilitate dispersion in the liquid medium.

The dosage of the active ingredient of the composition will, of course, vary with the nature of the severity of the condition to be treated and with the particular compound in the composition and its route of administration. It will also vary according to the age, weight and response of the individual patient.

In the embodiments where the pharmaceutical composition comprises trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate] hemipentahydrate (LCZ696) in the pharmaceutical compositions for use in the context of the present invention, the unit dose of the therapeutic agents sacubitril and valsartan together will be in the range from about 1 to about 1000 mg, such as 40 mg to 400 mg (e.g., 50 mg, 100 mg, 200 mg, 400 mg) per day. Alternatively lower doses may be given, for example doses of 0.5 to 100 mg; 0.5 to 50 mg; or 0.5 to 20 mg per day. As explanatory note, a unit dose of 100 mg LCZ696 delivering 100 mg of the two agents sacubitril and valsartan corresponds to 113.1 mg of trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate. Correspondingly, a unit dose of 200 mg requires 226.2 mg, and a unit dose of 400 mg requires 452.4 mg of trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate.

Dosages of the sum of the individual compounds (i)/(ii) in the combination of the pharmaceutical composition will be in the range from about 1 to about 1000 mg, such as 40 mg to 400 mg and include but are not limited to 5 mg, 20 mg, 25 mg, 40 mg, 50 mg, 80 mg, 100 mg, 200 mg, 400 mg, 800 mg and 1000 mg. Such dosages for compounds (i)/(ii) can be considered therapeutically effective amounts or dosage strengths. Ratios for the amount of each compound in the pharmaceutical composition are preferably in the about 1:1 molar ratio to achieve an optimal renal protection while still providing cardiovascular benefits. In preferred embodiments, the dosages of the individual compounds (i)/(ii) correspond to the same molecular amounts as in a pharmaceutical composition comprising a 50 mg, 100 mg, 200 mg or 400 mg dose of LCZ696. E.g. a 200 mg dose of LCZ696 corresponds approximately to 103 mg valsartan and 97 mg of sacubitril Pharmaceutical compositions containing a compound of formula(I) (such as compound LCZ696), or compounds (i)/(ii) can be administered any number of times per day, i.e. once a day (q.d.), twice (b.i.d.), three times, four time, etc. in an immediate release formation or less frequently as an extended or sustained release formation. Preferably the pharmaceutical composition is administered twice daily (b.i.d.). Corresponding doses may be taken, for example, in the morning, at mid-day or in the evening.

### Numbered Embodiments:

Embodiment 1. Use of
a) a therapeutically effective amount of the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a therapeutically effective amount of a 1:1 molar ratio of
   (i) valsartan or a pharmaceutically acceptable salt thereof; and
   (ii) sacubitril or a pharmaceutically acceptable salt thereof,
for the preparation of a medicament for the prevention or delaying time to first occurrence of mortality and/or of cardiovascular hospitalizations in a patient suffering from chronic systolic heart failure.

Embodiment 2. Use according to Embodiment 1, wherein the patient suffers from chronic systolic heart failure with reduced ejection fraction.

Embodiment 3. Use according to Embodiment 1 or 2, wherein the patient is a human patient.

Embodiment 4. Use according to any one of the preceding Embodiments 1 to 3, wherein the patient has at least one of the following characteristics
i) heart failure of NYHA class II, III or IV,
ii) an elevated plasma BNP or NT-proBNP level, preferably a plasma BNP ≥100 pg/mL (or NT-proBNP ≥400 pg/mL), more preferably a plasma BNP ≥150 pg/mL or NT-proBNP ≥600 pg/mL, and
iii) a reduced left ventricular ejection fraction (LVEF) of ≤40%, preferably ≤35%.

Embodiment 5. Use according to any one of the preceding Embodiments 1 to 4, wherein the patient suffers from chronic heart failure classified as NYHA class II, III or IV and has systolic dysfunction, preferably wherein the patient has a reduced left ventricular ejection fraction (LVEF) of ≤35%.

Embodiment 6. Use according to any one of the preceding Embodiments 1 to 5, wherein the patient suffers from chronic heart failure classified as NYHA class II, more preferably wherein the patient suffers from chronic heart failure classified as NYHA class II and has systolic dysfunction, preferably wherein the patient has a reduced left ventricular ejection fraction (LVEF) of ≤35%.

Embodiment 7. Use according to any one of Embodiments 1 to 6, wherein the medicament is for delaying time to first occurrence of cardiovascular hospitalization, preferably wherein the cardiovascular hospitalization is hospitalization for heart failure, non-fatal myocardial infarction, non-fatal stroke, or resuscitated sudden death, in particular hospitalization for heart failure, more particularly first hospitalization for worsening heart failure.

Embodiment 8. Use according to any one of Embodiments 1 to 7, wherein the medicament is for delaying time to the occurrence of mortality, in particular cardiovascular mortality.

Embodiment 9. Use according to Embodiment 8, wherein the mortality is a sudden death.

Embodiment 10. Use according to any one of Embodiments 1 to 9, wherein the medicament has been shown to reduce the rate of cardiovascular death and/or heart failure hospitalization.

Embodiment 11. Use according to Embodiments 10, wherein the medicament has been shown to reduce the rate of cardiovascular death and/or heart failure hospitalization in patients with chronic heart failure classified as NYHA class II, III or IV with systolic dysfunction, preferably wherein the patient also has a reduced left ventricular ejection fraction (LVEF) of ≤35%.

Embodiment 12. Use according to any one of Embodiments 1 to 9, wherein the medicament has been shown to reduce the rate of all-cause mortality.

Embodiment 13. Use of
a) a therapeutically effective amount of the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a therapeutically effective amount of a 1:1 molar ratio of
   (i) valsartan or a pharmaceutically acceptable salt thereof; and
   (ii) sacubitril or a pharmaceutically acceptable salt thereof,
for the preparation of a medicament for reducing the rate of cardiovascular death and/or heart failure hospitalization in patients with chronic heart failure with systolic dysfunction.

Embodiment 14. Use of
a) a therapeutically effective amount of the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a therapeutically effective amount of a 1:1 molar ratio of
   (i) valsartan or a pharmaceutically acceptable salt thereof; and
   (ii) sacubitril or a pharmaceutically acceptable salt thereof,
for the preparation of a medicament for the treatment of chronic heart failure in a patient with systolic dysfunction, wherein the medicament has been shown to reduce the rate of cardiovascular death and/or heart failure hospitalization in patients with chronic heart failure with systolic dysfunction.

Embodiment 15. Use according to Embodiment 13 or 14, wherein the patient has chronic heart failure classified as NYHA class II, III or IV.

Embodiment 16. Use according to any one of Embodiments 13 to 15, wherein the patient has a reduced left ventricular ejection fraction (LVEF) of ≤35%.

Embodiment 17. Use according to any one of Embodiments 13 to 16, wherein the medicament has been shown to reduce the rate of all-cause mortality.

Embodiment 18. Use according to any one of the preceding Embodiments 1 to 17, wherein the patient receives a base treatment with a stable dose of a beta-blocker, an aldosterone antagonists, and/ or a diuretic.

Embodiment 19. Use according to Embodiment 18, wherein the patient receives base treatment with a stable dose of a beta-blocker and optionally an aldosterone antagonist.

Embodiment 20. Use according to any one of the preceding Embodiments 1 to 19, wherein the patient, prior to receiving the medicament, is required to be on a stable dose of an angiotensin receptor blocker (ARB) or ACE inhibitor.

Embodiment 21. Use according to Embodiment 20, wherein the patient has to stop taking the angiotensin receptor blocker (ARB) or the ACE inhibitor at least 24 hours, preferably 36 hours, prior to taking the medicament.

Embodiment 22. Use according to any one of the preceding Embodiments 1 to 21, wherein the medicament is more effective than a medicament comprising a therapeutically effective amount of an ACE inhibitor.

Embodiment 23. Use according to Embodiment 22, wherein the ACE inhibitor is enalapril.

Embodiment 24. Use according to Embodiment 23, wherein the medicament is at least 10%, preferably at least 15% more effective than a medicament comprising a therapeutically effective amount of enalapril in delaying time to first occurrence of mortality, in particular cardiovascular death, and/or of cardiovascular hospitalizations, in particular hospitalizations for heart failure.

Embodiment 25. Use according to any one of the preceding Embodiments 1 to 23, wherein the medicament has been shown to be at least 10%, preferably at least 15% more effective in comparison to a medicament comprising a therapeutically effective amount of enalapril in reducing the rate of mortality, in particular cardiovascular death, and/or of cardiovascular hospitalizations, in particular hospitalizations for heart failure.

Embodiment 26. Use according to any one of the preceding Embodiments 1 to 23, wherein the medicament has been shown to be statistically superior to a medicament comprising a therapeutically effective amount of enalapril in reducing the rate of cardiovascular death and/or of cardiovascular hospitalizations, in particular hospitalizations for heart failure.

Embodiment 27. Use according to Embodiment 26, wherein the statistical superiority is expressed as a 20% risk reduction rate.

Embodiment 28. Use of
a) a therapeutically effective amount of the compound of the formula

   [(A₁)(A₂)](Na)_{y} • x H₂O (I)

   wherein
   A₁ is S-N-valeryl-N-{[2'-(1H-tetrazole-5-yl)-biphenyl-4-yl]-methyl}-valine in its anionic form;
   A₂ is (2R,4S)-5-biphenyl-4-yl-4-(3-carboxy-propionylamino)-2-methyl-pentanoic acid ethyl ester in its anionic form;
   Na is a sodium ion;
   y is 1 to 3; and
   x is 0 to 3;
   or
b) a combination comprising a therapeutically effective amount of a 1:1 molar ratio of
   (i) valsartan or a pharmaceutically acceptable salt thereof; and
   (ii) sacubitril or a pharmaceutically acceptable salt thereof,
for the preparation of a medicament for the treatment of chronic heart failure in a patient with systolic dysfunction, wherein the medicament has been shown to reduce the rate of cardiovascular death and/or heart failure hospitalization in patients with chronic heart failure with systolic dysfunction when compared to enalapril.

Embodiment 29. Use according to Embodiment 28, wherein the patient has chronic heart failure classified as NYHA class II, III or IV, in particular wherein the patient has chronic heart failure classified as NYHA class II, III or IV and has a reduced left ventricular ejection fraction (LVEF) of ≤35%.

Embodiment 30. Use according to Embodiment 28 or 29, wherein in comparison to enalapril there is an at least 20% risk reduction rate.

Embodiment 31. Use according to any one of Embodiments 28 to 30, wherein the medicament also has been shown to reduce the rate of all-cause mortality.

Embodiment 32. Use according to any one of Embodiments 22 to 31, wherein the medicament shows at least 20% risk reduction compared to a medicament comprising a therapeutically effective amount of enalapril with regard to the rate of cardiovascular death.

Embodiment 33. Use according to any one of Embodiments 22 to 32, wherein the medicament shows an at least 13% risk reduction compared to a medicament comprising a therapeutically effective amount of enalapril in reducing the rate of cardiovascular hospitalizations.

Embodiment 34. Use according to any one of Embodiments 22 to 33, wherein the medicament shows an at least 20% risk reduction compared to a medicament comprising a therapeutically effective amount of enalapril with regard to the rate of hospitalizations for heart failure, more particularly with regard to the rate of first hospitalization for worsening heart failure.

Embodiment 35. Use according to any one of Embodiments 22 to 34, wherein the medicament shows an at least 15% risk reduction compared to a medicament comprising a therapeutically effective amount of enalapril with regard to the rate of all-cause mortality.

Embodiment 36. Use according to any one of the preceding Embodiments 22 to 35, wherein the medicament has been shown to improve the NYHA class of more patients taking the medicament than of patients taking a medicament comprising a therapeutically effective amount of enalapril.

Embodiment 37. Use according to any one of the preceding Embodiments 22 to 36, wherein the medicament compared to a medicament comprising a therapeutically effective amount of enalapril has been shown to lead to at least one of the following:
- a greater decrease in NTproBNP levels,
- a consistent elevation in cGMP levels, or
- a greater reduction in troponin levels.

Embodiment 38. Use according to any one of the preceding Embodiments 22 to 37, wherein the medicament compared to a medicament comprising a therapeutically effective amount of enalapril showed a more potent blood pressure lowering effect.

Embodiment 39. Use according to any one of the preceding Embodiments 1 to 38, wherein a compound of formula (I) is used and the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

Embodiment 40. Use according to Embodiment 39, wherein the medicament comprises LCZ696 and is to be administered orally at a daily dose that may reach 400 mg, taken in one or more separate intakes.

Embodiment 41. Use according to Embodiment 40, wherein the medicament comprises LCZ696 and is to be administered orally at a daily dose that may reach 400 mg, taken in two separate intakes (b.i.d.).

Embodiment 42. Use according to Embodiment 40 or 41, wherein the medicament comprises LCZ696 and is to be administered orally at a starting dose of 100 mg b.i.d. for one to two weeks and afterwards up-titrated to 200 mg b.i.d..

Embodiment 43. Use according to any one of Embodiments 40 to 42, wherein the medicament is a pharmaceutical composition comprising LCZ696 and one or more pharmaceutically acceptable carriers, wherein LCZ696 is present in an amount of 50 mg, 100 mg or 200 mg per dosage unit of the pharmaceutical composition.

Embodiment 44. Use according to any one of the preceding Embodiments 1 to 38, wherein the combination comprising a therapeutically effective amount of a 1:1 molar ratio of
(i) valsartan or a pharmaceutically acceptable salt thereof; and
(ii) sacubitril or a pharmaceutically acceptable salt thereof,
is used for the preparation of the medicament.

Embodiment 45. Use according to Embodiment 44, wherein the combination is to be administered orally in two separate intakes (b.i.d.) per day, each intake comprising 103 mg valsartan, or a pharmaceutically acceptable salt thereof, and 97 mg sacubitril, or a pharmaceutically acceptable salt thereof, per dosage unit.

The following example is illustrative, but does not serve to limit the scope of the invention described herein.

### Example 1:

A randomized, double-blind, parallel group, active-controlled, two-arm, event-driven trial comparing the long-term efficacy and safety of enalapril and LCZ696 on morbidity and mortality in patients with chronic symptomatic heart failure and reduced ejection fraction (HF-REF) [PARADIGM-HF].

### LCZ696:

LCZ696 refers to the supramolecular complex trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate. This compound and pharmaceutical compositions thereof have been previously disclosed in WO2007/056546 and WO 2009/061713, whose preparative teachings are incorporated herein by reference.

LCZ696 is a first-in-class angiotensin receptor neprilysin inhibitor that comprises the molecular moieties of the NEP (neutral endopeptidase EC 3.4.24.11) inhibitor pro-drug sacubitril (INN, also known as AHU377 and N-(3-carboxy-1-oxopropyl)-(4S)-p-phenylphenylmethyl)-4-amino-(2R)-methylbutanoic acid ethyl ester) and the angiotensin receptor blocker valsartan as a single compound. Sacubitril is metabolized by enzymatic cleavage to LBQ657 (N-(3-carboxy-1-oxopropyl)-(4S)-p-phenylphenylmethyl)-4-amino-(2R)-methylbutanoic acid), the active inhibitor of neutral endopeptidase, which is the major enzyme responsible for the breakdown of atrial natriuretic peptides.

### Enalapril

The ACE inhibitor Enalapril or (2S)-1-[(2S)-2-{[(2S)-1-ethoxy-1-oxo-4-phenylbutan-2-yl]amino}propanoyl]-pyrrolidine-2-carboxylic acid can be purchased from commercial sources or can be prepared according to known methods.

### Objective & Methods:

Patients with chronic HF, NYHA functional class II-IV symptoms, an elevated plasma B-type natriuretic peptide (BNP) or NT-proBNP level and, initially, a left ventricular ejection fraction of ≤40% (later amended to ≤35%) are eligible. Patients enter a single blind enalapril run-in period (titrated to 10 mg bid) which, depending on tolerability, is followed by an LCZ696 run-in period (100 mg titrated to 200 mg bid). Then, patients tolerating both drugs at the target dose, are randomized 1:1 to either enalapril 10 mg bid or LCZ696 200 mg bid. The primary outcome is the composite of cardiovascular death or HF hospitalization, although the trial is powered to detect a 15% relative risk reduction in cardiovascular death with LCZ696, compared with enalapril. Secondary outcome measures are change in the Kansas City Cardiomyopathy Questionnaire (KCCQ) clinical summary score at 8 months, change in renal function, and time to all-cause mortality.

### Detailed Study Design and Procedures

### TRIAL DESIGN AND METHODS

PARADIGM-HF is a randomized, double-blind, parallel group, active-controlled, two-arm, event-driven trial comparing the long-term efficacy and safety of enalapril and LCZ696 in patients with chronic symptomatic heart failure and reduced ejection fraction (HF-REF). The trial was designed by the academic members of the Executive Committee (EC) in collaboration with Novartis personnel.

### Patients

Entry criteria: 1) age 18 years or older and able to give written informed consent 2) New York Heart Association (NYHA) functional class II - IV, 3) left ventricular ejection fraction (LVEF) ≤ 35% (initially this was ≤ 40% but changed in a protocol amendment dated December 15, 2010), 4) plasma B-type natriuretic peptide (BNP) ≥ 150 pg/ml (or N-terminal [NT]-proBNP ≥ 600 pg/ml) at the screening visit or a BNP ≥ 100 pg/mL (or NT-proBNP ≥ 400 pg/ml) and a hospitalization for HF within the last 12 months, 5) treatment with a stable dose of an ACE inhibitor or an ARB equivalent to enalapril 10 mg/d for at least 4 weeks before the screening visit, 6) treatment with stable dose of a β-blocker for at least 4 weeks prior to Visit 1, unless contraindicated or not tolerated. Although not required, the protocol specified that an aldosterone antagonist should also be considered in all patients, taking account of renal function, serum potassium and tolerability. If given, the dose of aldosterone antagonist should be stable for at least 4 weeks prior to the screening visit.

### The key exclusion criteria include:

1. History of hypersensitivity or allergy to any of the study drugs, drugs of similar chemical classes, ACE inhibitors (ACEIs), angiotensin receptor blockers (ARBs) or neprilysin inhibitors as well as known or suspected contraindications to the study drugs.
2. Previous history of intolerance to recommended target doses of ACEls or ARBs.
3. Known history of angioedema.
4. Requirement of treatment with both ACEls and ARBs.
5. Current acute decompensated heart failure (exacerbation of chronic heart failure manifested by signs and symptoms that may require intravenous therapy).
6. Symptomatic hypotension and/or a systolic blood pressure (SBP) < 100 mmHg at Visit 1 (screening) or < 95 mmHg at Visit 3 or at Visit 5 (randomization).
7. Estimated GFR (eGFR) < 30 mL/min/1.73m2 at Visit 1 (screening), Visit 3 (end of enalapril run-in), or Visit 5 (end of LCZ696 run-in and randomization) or > 35% decline in eGFR between Visit 1 and Visit 3 or between Visit 1 and Visit 5.
8. Serum potassium > 5.2 mmol/L at Visit 1 (screening) or > 5.4 mmol/L at Visit 3 or Visit 5 (randomization).
9. Acute coronary syndrome, stroke, transient ischemic attack, cardiac, carotid or other major CV surgery, percutaneous coronary intervention or carotid angioplasty within the 3 months prior to Visit 1.
10. Coronary or carotid artery disease likely to require surgical or percutaneous intervention within the 6 months after Visit 1.
11. Implantation of a cardiac resynchronization therapy device (CRT) within 3 months prior Visit 1 or intent to implant a CRT.
12. History of heart transplant or on a transplant list or with left ventricular assistance device.
13. History of severe pulmonary disease.
14. Diagnosis of peripartum or chemotherapy induced cardiomyopathy within the 12 months prior to Visit 1.
15. Documented untreated ventricular arrhythmia with syncopal episodes within the 3 months prior to Visit 1.
16. Symptomatic bradycardia or second or third degree atrio-ventricular block without a pacemaker.
17. Presence of haemodynamically significant mitral and/or aortic valve disease, except mitral regurgitation secondary to left ventricular dilatation.
18. Presence of other haemodynamically significant obstructive lesions of left ventricular outflow tract, including aortic and sub-aortic stenosis.
19. Any surgical or medical condition which might significantly alter the absorption, distribution, metabolism, or excretion of study drugs, including but not limited to any of the following:
   - History of active inflammatory bowel disease during the 12 months before Visit 1.
   - Active duodenal or gastric ulcers during the 3 months prior to Visit 1.
   - Evidence of hepatic disease as determined by any one of the following: aspartate aminotransferase or alanine aminotransferase values exceeding 2 x upper limit of normal at Visit 1, history of hepatic encephalopathy, history of oesophageal varices, or history of porto-caval shunt.
   - Current treatment with cholestyramine or colestipol resins.
20. Presence of any other disease with a life expectancy of < 5 years.

### STUDY DESIGN

The study consists of four phases: 1) screening, 2) single-blind enalapril run-in, 3) and single-blind LCZ696 run-in, and 4) randomized double-blind treatment.

### Screening (Visit 1)

At the screening visit, patient eligibility was assessed according to the inclusion/exclusion criteria. Any local measurement of LVEF ≤35%, made within the past 6 months, was acceptable provided there was no subsequent LVEF measurement above 35%. Eligibility BNP (and NT-proBNP), serum potassium and estimated glomerular filtration rate (eGFR) were measured in a central laboratory.

### Enalapril Active Run-in Period (Visit 2)

At Visit 2, most eligible patients started two weeks of single-blind treatment with enalapril 10 mg bid. A lower dose of enalapril (5 mg bid) was allowed for patients currently treated with an ARB and for those taking a low dose of ACE inhibitor if the investigator was concerned that switching directly to enalapril 10 mg bid might not be tolerated (e.g. because of hypotension, renal dysfunction, and/or hyperkalaemia). These patients were up-titrated to enalapril 10 mg bid after 1-2 weeks. Patients tolerating enalapril 10 mg bid were eligible for visit 3.

### LCZ696 Active Run-in Period (Visits 3 and 4)

At visit 3, patients started single-blind treatment with LCZ696 100 mg bid. After 1-2 weeks the dose was up-titrated to 200 mg bid, for a further 2-4 weeks.

Other heart failure medication (except for an ACE inhibitor or ARB) was continued during the run-in periods.

### Randomization to double-blind treatment (Visit 5)

Patients tolerating both enalapril 10 mg bid and LCZ696 200 mg bid, were randomized in a 1:1 ratio to double-blind treatment with either enalapril 10 mg bid or LCZ696 200 mg bid. Study visits occur every 2 to 8 weeks during the first 4 months of the double-blind period and every 4 months thereafter (with additional unscheduled visits, at the discretion of the investigator).

There were two short washout periods during the run-in periods to minimize the potential risk of angioedema due to overlapping ACE inhibition and NEP inhibition at Visit 3 and Visit 5: (i) enalapril was stopped a day prior to starting LCZ-696 at visit3 and (ii) LCZ696 was stopped a day prior to starting randomized study drug at Visit 5.

### Monitoring of safety and tolerability during double blind period

Patients are assessed at each study visit for hyperkalaemia, symptomatic hypotension, increase in serum creatinine, angioedema and other AEs and serious AEs (SAEs). Patients who can no longer tolerate the target dose of study drug can be down-titrated to the lower dose at the investigator's discretion (after considering whether any other relevant non disease-modifying therapy can be discontinued, e.g. a calcium channel or alpha-adrenoceptor blocker in a hypotensive patient). The dose of background disease modifying drugs, such as β-blockers, should not be reduced to facilitate maintenance of study drug. Every attempt should be made to re-challenge the patients so as to maintain as many patients as possible on the target dose of study drug.

### Collection and adjudication of potential angioedema events

Potential angioedema cases are identified in two ways: (1) proactive reporting of any events that resemble angioedema by site investigators, and (2) routine safety monitoring by the sponsor for signs or symptoms suggestive of potential angioedema. All identified cases are submitted to an independent angioedema adjudication committee for a final decision.

### STUDY OBJECTIVES

### Primary objectives

The purpose of this study was to evaluate the effect of LCZ696 200 mg bid compared with enalapril 10 mg bid, in addition to conventional heart failure treatment, in delaying time to first occurrence of either cardiovascular death or heart failure hospitalization.

### Secondary objectives

To test whether LCZ696, compared to enalapril, is superior:
- in improving the Kansas City Cardiomyopathy Questionnaire (KCCQ) clinical summary score at 8 months.
- in delaying the time to all-cause mortality.
- in delaying the time to first occurrence of either: i) a 50% decline in estimated glomerular filtration rate (eGFR) relative to baseline (i.e., Visit 5), ii) >30 mL/min/1.73 m2 decline in eGFR relative to baseline , or (iii) reaching end stage renal disease.

### STATISTICAL CONSIDERATIONS

The estimated annual event rate for the primary endpoint in the control (enalapril) arm of PARADIGM-HF was based on the Candesartan in Heart failure: Assessment of Reduction in Mortality and morbidity (CHARM)-Added trial where the annual event rate for the same composite was 16.6% in the placebo group (and 14.1% in the candesartan group). Because patients are treated with a higher dose of ACE inhibitor (or LCZ696 equivalent) in PARADIGM-HF and a greater proportion were expected to receive a beta-blocker and a mineralocorticoid antagonist, a more conservative expected annual event rate of 14.5% was chosen for sample size calculation. The annual CV mortality rate of 7% was estimated from the CHARM-Added trial in a similar manner. We also anticipated that the requirement for an elevated BNP or NT proBNP level at enrolment would ensure an adequate event rate.

The sample size is based upon the CV mortality (although the trial will continue until the required number of patients have experienced CV death or heart failure hospitalization - see below). A total of 1,229 CV deaths are required to give 80% power to detect a relative risk reduction of 15% in the LCZ696 group, compared with the enalapril group. A total of approximately 8000 patients are required to accrue this number of events, assuming an annual CV death rate of 7% in the enalapril group, with a mean follow up of approximately 34 months.

Assuming an annual rate of CV death or heart failure hospitalization in the enalapril group of 14.5%, and the same sample-size and follow-up period, at least 2,410 patients are expected to experience a primary event. This means that PARADIGM-HF should have more than 97% power to detect a relative risk reduction of 15% in this composite.

A final statistical analysis plan will be developed prior to the end of the study and treatment unblinding.

### STUDY DURATION, INTERIM ANALYSES AND EARLY TERMINATION

As PARADIGM-HF is an event-driven trial, all randomized patients will remain in the trial until 2410 patients have experienced a CV death or heart failure hospitalization, unless the DMC recommends that the study be stopped earlier for efficacy or safety reasons. The total length of the trial will depend on the duration of the patient recruitment period and the time taken to accrue the pre-specified number of patients with a primary event. Currently, three interim efficacy analyses are planned when approximately one third, one half, and two thirds of the primary events have occurred. The one-sided significance level of α to be used for the final analysis will be adjusted for the interim efficacy analyses to control the overall type I error at 2.5% (one-sided). A futility analysis will also be performed at each of these interim analyses, permitting early termination of the trial for lack of efficacy.

### SAFETY CONSIDERATIONS

In view of the limited exposure of patients with HF to LCZ696 before the start of PARADIGM-HF, the DMC performed a safety assessment after the first 100, 300, and 600 patients completed the run-in period and the number of patients exposed to study drug was also limited to 600 until the DMC had evaluated safety in the first 200 patients to complete 4 weeks of double-blind therapy. Thus, this study employed a seamless design, incorporating "Phase II" into a "Phase III" outcomes trial, as considered further in the Discussion. Further interim safety assessments are planned twice a year.

### Reference

Detailed study design and procedures can be found under www.clinicaltrials.gov, study number NCT01035255, and as published in The European Journal of Heart Failure by McMurray et al (18th April 2013) titled "Dual angiotensin receptor and neprilysin inhibition as an alternative to angiotensin converting enzyme inhibition in patients with chronic systolic heart failure: rationale for and design of the Prospective comparison of ARNI with ACEI to Determine Impact on Global Mortality and morbidity in Heart Failure trial (PARADIGM-HF)", references which are herewith incorporated by reference.

### RESULTS

### SUMMARY:

The trial was stopped early, according to prespecified rules, after a median follow-up of 27 months. The primary outcome occurred in 914 (21.8%) of patients in the LCZ696 group compared with 1117 (26.5%) in the enalapril group (hazard ratio 0.80; 95% confidence interval [Cl], 0.73-0.87; P<0.0001). A total of 711 (17.0%) of patients receiving LCZ696 and 835 (19.8%) receiving enalapril died from any cause (hazard ratio 0.84; 95% Cl, 0.76-0.93; P<0.001); of these, 558 (13.3%) and 693 (16.5%), respectively, died from cardiovascular causes (hazard ratio 0.80; 95% Cl, 0.71-0.89; P<0.0001). LCZ696 also reduced hospitalization for heart failure by 21% (P<0.001) and improved the symptoms and physical limitations of heart failure (P=0.001) more than enalapril. The LCZ696 group had more hypotension and non-serious angioedema but less renal impairment, hyperkalemia and cough than the enalapril group.

### DETAILS

### Study Patients

From December 8, 2009 through January 17, 2013, 10,521 patients at 1043 centers in 47 countries entered the run-in period, of whom 2079 patients did not fulfill the criteria for randomization and 43 patients were randomized erroneously or enrolled at sites that were closed due to serious Good Clinical Practice violations and were prospectively omitted (before the trials' end) from all analyses; accordingly, 4187 were randomly assigned to LCZ696 and 4212 to enalapril for the intention-to-treat analysis (Figure 1). The groups were balanced with respect to baseline characteristics; patients were well treated with recommended pharmacological therapy for chronic heart failure (Table 1).

**Table 1. Baseline Characteristics of the Patients, According to Study Group***

| | | **LCZ696 (n=4187)** | **Enalapril (n=4212)** |
|---|---|---|---|
| **Aqe (years)** | | 63.8 ± 11.5 | 63.8 ± 11.3 |
| **Female sex (%)** | | 879 (21.0) | 953 (22.6) |
| **Race** | | | |
| | Caucasian | 2763 (66.0%) | 2781 (66.0%) |
| | Black | 213 (5.1%) | 215 (5.1%) |
| | Asian | 759 (18.1%) | 750 (17.8%) |
| | Other | 452 (10.8%) | 466 (11.1%) |
| **Region** | | | |
| | North America | 310 (7.4%) | 292 (6.9%) |
| | Latin America | 713 (17.0%) | 720 (17.1%) |
| | Western Europe | 1026 (24.5%) | 1025 (24.3%) |
| | Central Europe | 1393 (33.3%) | 1433 (34.0%) |
| | Asia/Pacific | 745 (17.8%) | 742 (17.6%) |
| **Cause of heart failure** | | | |
| | Ischemic cardiomyopathy | 2506 (59.9%) | 2530 (60.1%) |
| | Nonischemic cardiomyopathy | 1681 (40.1%) | 1682 (39.9%) |
| **Time since diagnosis of heart failure** | | | |
| | Less than 1 year | 1275 (30.5%) | 1248 (29.6%) |
| | 1-5 years | 1621 (38.7%) | 1611 (38.2%) |
| | More than 5 years | 1291 (30.8%) | 1353 (32.1%) |
| **LV ejection fraction (%)** | | 29.6 ± 6.1 | 29.4 ± 6.3 |
| | Less than or equal to 35% | 3715 (88.7%) | 3722 (88.4%) |
| | More than 35% | 472 (11.3%) | 489 (11.6%) |
| **NYHA functional class** | | | |
| | Class I | 180 (4.3%) | 209 (5.0%) |
| | Class II | 2998 (71.6%) | 2921 (69.4%) |
| | Class III | 969 (23.1%) | 1049 (24.9%) |
| | Class IV | 33 (0.8%) | 27 (0.6%) |
| | Missinq | 7 (0.2%) | 6 (0.1%) |
| **Physiological measures** | | | |
| | Systolic blood pressure (mm Hg) | 122 ± 15 | 121 ± 15 |
| | Diastolic blood pressure (mm Hq) | 74 ± 10 | 74 ± 10 |
| | Heart rate (beats/min) | 72 ± 12 | 73 ± 12 |
| **Body mass index (kg/m²) ‡** | | 28.1 ± 5.5 | 28.2 ± 5.5 |
| **Laboratory measures** | | | |
| | N-terminal pro-B-type natriuretic peptide (pq/ml) | 1631 (885-3154) | 1594 (886-3305) |
| | B-type natriuretic peptide (pq/ml) | 255 (155-474) | 251 (153-465) |
| | Serum potassium (mmol/l) | 4.5 ± 0.4 | 4.5 ± 0.4 |
| | Serum creatinine (mq/dl) | 1.13 ± 0.3 | 1.12 ± 0.3 |
| | Estimated GFR (ml/min/1.73m²) | 68 ± 20 | 68 ± 20 |
| **Medical history** | | | |
| | Hypertension | 2969 (71%) | 2971 (71%) |
| | Diabetes | 1451 (35%) | 1456 (35%) |
| | Atrial fibrillation | 1517 (36.2%) | 1574 (37.4%) |
| | Hospitalization for heart failure | 2607 (62.3%) | 2667 (63.3%) |
| | Myocardial infarction | 1818 (43.4%) | 1816 (43.1%) |
| | Stroke | 355 (8.5%) | 370 (8.8%) |
| | Coronary artery bypass surgery | 686 (16.4%) | 617 (14.6%) |
| | Percutaneous coronary intervention | 901 (21.5%) | 900 (21.4%) |
| **Pre-trial use of angiotensin converting enzyme inhibitor/angiotensin receptor blocker** | | | |
| | Angiotensin converting enzyme inhibitor | 3266 (78.0%) | 3266 (77.5%) |
| | Angiotensin receptor blocker | 929 (22.2%) | 963 (22.8%) |
| **Medications at randomization** | | | |
| | Diqitalis | 1223 (29.3%) | 1316 (31.2%) |
| | Diuretics | 3363 (80.3%) | 3375 (80.1%) |
| | Beta-adrenergic blockers | 3899 (93.1%) | 3912 (92.9%) |
| | Mineralocorticoid antagonists | 2271 (54.2%) | 2400 (57.0%) |
| | Oral anticoaqulant | 1299 (31.0%) | 1386 (32.9%) |
| | Antiplatelet drug | 2386 (57.0%) | 2350 (55.8%) |
| | Lipid lowering agent | 2370 (56.6%) | 2359 (56.0%) |
| **Devices for heart failure at screening visit** | | | |
| | Implantable cardioverter-defibrillator | 623 (14.9%) | 620 (14.7%) |
| | Cardiac resynchronization therapy | 292 (7.0%) | 282 (6.7%) |
| | Implantable cardioverter-defibrillator with cardiac resynchronization | 212 (5.1%) | 212 (5.0%) |

| | | | |
|---|---|---|---|
| * Plus-minus values are means ± SD. For natriuretic peptides, the values are shown as median and 25%/75% interquartile ranges. Percentages may not total 100 because of rounding. To convert the values for creatinine to micromoles per liter, multiply by 88.4. GFR denotes glomerular filtration rate. † Race was reported by the investigators; ‡ The body-mass index is the weight in kilograms divided by the square of the height in meters. The data for NYHA class reflects the status of patients at randomization; all patients were required to have at least class II symptoms at screening. The two groups were similar with respect to baseline characteristics | | | |

### Study Drug Administration and Follow-Up

Excluding death, the study drug was discontinued in 746 patients (17.8%) receiving LCZ696 and 833 patients (19.8%%) receiving enalapril (P=0.022). At the patient's last assessment among those taking the study medication, the mean (±SD) doses in the LCZ696 and enalapril groups were 375±71 mg and 18.9±3.4 mg, respectively. Eleven patients in the LCZ696 group and 9 patients in the enalapril group were lost to follow-up and censored at last contact. The median duration of follow-up was 27 months, with no difference between the groups.

### Study Outcomes

Death from cardiovascular causes or hospitalization for heart failure (the primary outcome) occurred in 914 patients (21.8%) in the LCZ696 group and 1117 patients (26.5%) in the enalapril group (Table 2); the hazard ratio in the LCZ696 group, compared with the enalapril group, was 0.80 (95% confidence interval [Cl], 0.73 to 0.87; P<0.0001). The difference in favor of LCZ696 was seen early in the trial and at each interim analysis. A total of 558 deaths (13.3%) in the LCZ696 group and 693 (16.5%) in the enalapril group were due to cardiovascular causes (hazard ratio 0.80; 95% Cl, 0.71 to 0.89; P<0.0001) (Table 2). Of the patients receiving LCZ696, 537 (12.8%) were hospitalized for heart failure, compared with 658 patients (15.6%) receiving enalapril (hazard ratio 0.79; 95% Cl, 0.71 to 0.89; P<0.0001) (Table 2). Over the duration of the trial, the number of patients needed to treat to prevent one primary event and one cardiovascular death was 21 and 32, respectively. A total of 711 patients (17.0%) in the LCZ696 group and 835 patients (19.8%) in the enalapril group died for any reason (hazard ratio 0.84; 95% Cl, 0.76 to 0.93; P<0.001) (Table 2). The effect of LCZ696 was consistent across all prespecified subgroups; a nominally significant interaction between NYHA class at randomization and the effect of treatment on the primary endpoint (P=0.034, unadjusted for multiple comparisons) was not seen for cardiovascular mortality (P=0.76).

The mean change from baseline to month 8 in the KCCQ clinical summary score was - 2.99 in the LCZ696 group and -4.63 in the enalapril group (between-group difference 1.64; 95% Cl, 0.63 to 2.65; P=0.001). When zero values were not imputed for patients who died, the score improved in the LCZ696 group and declined in the enalapril group; the between-group difference (0.95, 95% Cl 0.31, 1.59) remained significant (P=0.004). New onset atrial fibrillation developed in 84 patients in the LCZ696 group and 83 patients in the enalapril group (P=0.84), Table 2. A total of 94 patients in the LCZ696 group and 108 patients in the enalapril group experienced protocol-defined decline in renal function (P=0.28), Table 2; 8 in the LCZ696 group and 16 in the enalapril group progressed to end-stage renal disease (P=0.11).

**Table 2. Protocol-Specified Primary and Secondary Outcomes**

| **Table 2. Protocol-specified Primary and Secondary Outcomes** | | | | | |
|---|---|---|---|---|---|
| **Outcome** | | **LCZ696 (N = 4187)** | **Enalapril (N = 4212)** | **Hazard Ratio (95% Cl)** | **P Value** |
| **Primary composite outcome** | | | | | |
| Death from a cardiovascular causes or first hospitalization for worsening heart failure | | 914 (21.8) | 1117 (26.5) | 0.80 (0.73-0.87) | < 0.0001 |
| | Death from cardiovascular causes | 558 (13.3) | 693 (16.5) | 0.80 (0.71-0.89) | < 0.0001 |
| | First hospitalization for worsening heart failure | 537 (12.8) | 658 (15.6) | 0.79 (0.71-0.89) | < 0.0001 |
| **Secondary outcomes** | | | | | |
| Death from any cause | | 711 (17.0) | 835 (19.8) | 0.84 (0.76-0.93) | < 0.001 |
| New onset atrial fibrillation* | | 84/2670 (3.2) | 83/2638 (3.2) | 0.97 (0.72-1.31) | 0.84 |
| Decline in renal function** | | 94 (2.3) | 108 (2.6) | 0.86 (0.65-1.13) | 0.28 |
| | | | | | |
| Change in KCCQ Clinical Summary Score at 8 months^{†} | | -2.99 (± 0.36) | -4.63 (± 0.36) | 1.64 (0.63-2.65) | 0.001 |

| | | | | | |
|---|---|---|---|---|---|
| For all outcomes other than KCCQ, the data are shown as number of patients with an event as a fraction of number of patients at risk, and the treatment effect is displayed as the hazard ratio, with the 95% confidence interval in parentheses, which were calculated with the use of stratified Cox models. P values are two-sided and were calculated by means of a stratified log-rank test and were not adjusted for multiple comparisons. | | | | | |

### Safety

Four patients (2 in each group) did not start the study medication and were excluded from the safety analyses. During the run-in period, 12.5% withdrew for an adverse event (particularly cough, hyperkalemia, renal dysfunction and hypotension) and did so at a higher exposure-adjusted rate while taking enalapril than LCZ696. Following randomization, although the LCZ696 group was more likely to report symptomatic hypotension, these events rarely required the discontinuation of treatment. In contrast, compared with the enalapril group, the LCZ696 group was less likely to report cough as an adverse event or experience serum creatinine level ≥ 2.5 mg/dl or serum potassium level > 6.0 mmol/l (both P<0.05), Table 3. Overall, fewer patients in the LCZ696 group than in the enalapril group stopped their study medication for any adverse event (10.7% vs 12.3%, respectively, P=0.03) or because of renal impairment (0.7% vs 1.4%, respectively, P=0.002).

Compared with the value at randomization, the systolic blood pressure at 8 months was 3.2±0.4 mm Hg lower in the LCZ696 group than in the enalapril group, P<0.001 for the difference between groups, but when modeled as a time-dependent covariate, the difference in blood pressure was not a determinant of the incremental benefits of LCZ696. At 8 months, there were no significant differences in heart rate or serum creatinine from randomization between the two groups. Angioedema was confirmed by blinded adjudication in 19 patients in the LCZ696 group and 10 patients in the enalapril group (P=0.13). No patient had airway compromise or required mechanical airway protection.

### Discussion

These results demonstrate that inhibition of both the angiotensin II receptor and neprilysin with LCZ696 reduced the risk of cardiovascular death and the risk of hospitalization for heart failure more than ACE inhibition with enalapril in patients with chronic heart failure and reduced ejection fraction. LCZ696 also reduced the risk of death for any reason and improved symptoms and physical limitations of heart failure more than enalapril. The magnitude of these advantages of LCZ696 over ACE inhibition was highly statistically significant and clinically important, even though the drug was compared against enalapril given at a dose shown to reduce mortality when compared with placebo. The benefit of LCZ696 was seen in patients who were already receiving all other drugs known to improve survival in heart failure (i.e. beta-blockers and mineralocorticoid receptor antagonists); was consistent in all relevant subgroups with respect to cardiovascular mortality; and was apparent early in the trial.

In conclusion, the angiotensin receptor-neprilysin inhibitor LCZ696 reduced the risk of death and the risk of hospitalization for heart failure more than ACE inhibition alone. The magnitude of the advantage of LCZ696 over enalapril on cardiovascular mortality was at least as large as that of enalapril over placebo during long-term treatment. This robust finding provides strong support for using this new approach instead of ACE inhibitors or ARBs in the treatment of chronic heart failure.

### Conclusion

Based on compelling efficacy the trial was closed early since the combined primary endpoint was met: LCZ696 delayed cardiovascular death and reduced heart failure hospitalizations in patients with chronic heart failure with reduced ejection fraction in comparison to the current standard of care enalapril.

The results of PARADIGM-HF show that treatment with LCZ696 was superior to currently recommended doses of enalapril. This will have profound implications for the care of patients with chronic heart failure.

## Claims

1. A sugar-coated tablet, tablet, capsule, bar, sachet, granule, syrup, aqueous or oily suspension or suppository, or ampoule comprising
a) a therapeutically effective amount of the compound of the formula
[(A₁)(A₂)](Na)_{y} • x H₂O (I)
wherein
A₁ is S-N-valeryl-N-{[2'-(1H-tetrazole-5-yl)-biphenyl-4-yl]-methyl}-valine in its anionic form;
A₂ is (2R,4S)-5-biphenyl-4-yl-4-(3-carboxy-propionylamino)-2-methyl-pentanoic acid ethyl ester in its anionic form;
Na is a sodium ion;
y is 1 to 3; and
x is 0 to 3;
or
b) a combination comprising a therapeutically effective amount of a 1:1 molar ratio of
(i) valsartan or a pharmaceutically acceptable salt thereof; and
(ii) sacubitril or a pharmaceutically acceptable salt thereof,
for the use of preventing or delaying time to first occurrence of cardiovascular mortality and/or cardiovascular hospitalizations in a patient suffering from chronic systolic heart failure with reduced ejection fraction.

2. The pharmaceutical preparation for use according to Claim 1, wherein the use is delaying time to first occurrence of cardiovascular hospitalization wherein the cardiovascular hospitalization is hospitalization for heart failure, non-fatal myocardial infarction, non-fatal stroke, or resuscitated sudden death.

3. The pharmaceutical preparation for use according to Claim 2, wherein the use is delaying time to first occurrence of cardiovascular hospitalization for heart failure.

4. The pharmaceutical preparation for use according to any one of Claims 1-3, wherein the patient receives a base treatment with a stable dose of a beta-blocker.

5. The pharmaceutical preparation for use according to any one of Claims 1-4, wherein the pharmaceutical preparation comprises the compound of formula (I) which is trisodium [3-((1S,3R)-1-biphenyl-4-ylmethyl-3-ethoxycarbonyl-1-butylcarbamoyl)propionate-(S)-3'-methyl-2'-(pentanoyl{2" -(tetrazol-5-ylate)biphenyl-4'-ylmethyl}amino)butyrate] hemipentahydrate.

6. The pharmaceutical preparation for use according to Claim 5, wherein the pharmaceutical preparation is to be administered orally at a daily dose that may reach 400 mg, taken in one or more separate intakes.

7. The pharmaceutical preparation for use according to Claim 6, wherein the pharmaceutical preparation is to be administered orally at a daily dose that may reach 400 mg, taken in two separate intakes (b.i.d.).

8. The pharmaceutical preparation for use according to Claim 6 or Claim 7, wherein the pharmaceutical preparation is to be administered orally at a starting dose of 100 mg b.i.d. for one to two weeks and afterwards up-titrated to 200 mg b.i.d..

9. The pharmaceutical preparation for use according to any one of Claims 1-4, wherein the pharmaceutical preparation comprises the combination comprising a therapeutically effective amount of a 1:1 molar ratio of
(i) valsartan or a pharmaceutically acceptable salt thereof; and
(ii) sacubitril or a pharmaceutically acceptable salt thereof.

10. The pharmaceutical preparation for use according to Claim 9, wherein the combination is to be administered orally in two separate intakes (b.i.d.) per day, each intake comprising 103 mg valsartan, or a pharmaceutically acceptable salt thereof, and 97 mg sacubitril, or a pharmaceutically acceptable salt thereof, per dosage unit.

11. The pharmaceutical preparation for use according to any one of Claims 1-10, wherein the patient has at least one of the following additional characteristics
i) heart failure of NYHA class II, III or IV,
ii) an elevated plasma BNP or NT-proBNP level, preferably a plasma BNP ≥100 pg/ml (or NT-proBNP ≥400 pg/ml), more preferably a plasma BNP ≥150 pg/ml or NT-proBNP ≥600 pg/ml, and
iii) a reduced left ventricular ejection fraction (LVEF) of ≤40%

12. The pharmaceutical preparation for use according to Claim 11, wherein the patient has a reduced left ventricular ejection fraction (LVEF) of ≤35%.

13. The pharmaceutical preparation for use according to any one of Claims 1-12, wherein the pharmaceutical preparation is more effective in preventing or delaying time to first occurrence of cardiovascular mortality and/or cardiovascular hospitalizations in patients suffering from chronic systolic heart failure with reduced ejection fraction, compared to enalapril administered twice daily at a dose of 10 mg.

14. The pharmaceutical preparation for use according to Claim 13, wherein the pharmaceutical preparation is at least 15% more effective than a medicament comprising enalapril administered twice daily at a dose of 10 mg, in preventing or delaying time to first occurrence of cardiovascular mortality and/or cardiovascular hospitalizations.
